# EUROPÄISCHE PATENTSCHRIFT

(11) **EP 1 112 737 B1**
(45) Veröffentlichungstag und Bekanntmachung des Hinweises auf die Patenterteilung: **22.01.2003**
(21) Anmeldenummer: 00250443.9
(22) Anmeldetag: 19.12.2000
(51) Int. Cl.: A61K 9/20, A61K 31/165

(54) **Orale pharmazeutische Zubereitung enthaltend Paracetamol und Metoclopramid**
Oral pharmaceutical preparation comprising paracetamol and metoclopramide
Préparation pharmaceutique orale comprenant du Paracétamol et de la Métoclopramide

(30) Priorität: 21.12.1999 DE 19963319
(43) Veröffentlichungstag der Anmeldung: 04.07.2001
(73) Patentinhaber: Hennig Arzneimittel GmbH & Co. KG, 65439 Flörsheim (DE)
(72) Erfinder: Francas, Gernot, Dr., 67549 Worms (DE)
(74) Vertreter: Schubert, Klemens, Dr.

(56) Entgegenhaltungen:
- US-A- 4 325 971
- BECKER A., BERNER G., LEUSCHNER F., VÖGTLE-JUNKERT U.: "Pharmakokinetische aspekte zur kombination von metoclopramid und paracetamol" ARZNEIMITTELFORSCHUNG, Bd. 42, Nr. 4, 1992, Seiten 552-555, XP000926413
- MATTS S.G, BOSTON P.F: "Paracetamol plus metoclopramide ('Paramax') as an adjuct analgesic in the treatment of arthritis" CURRENT MEDICAL RESEARCH AND OPINION, Bd. 8, Nr. 8, 1983, Seiten 547-552, XP000926422

## Beschreibung

Die vorliegende Erfindung betrifft eine orale pharmazeutische Zubereitung umfassend Paracetamol und Metoclopramid in Kombination.

Die Verwendung von Paracetamol und Metoclopramid in Form von Oralia ist beispielsweise bekannt aus EP-0 011 490 oder EP-0 011 489. Hierbei werden Tabletten oder Brausepulver beschrieben, welche beide Wirkstoffe in einer innigen Vermischung enthalten.

Aus US4325971 ist eine oral zu verabreichende pharmazeutische Zubereitung mit Paracetamol und Metoclopramid bekannt.

Ausgehend von der Beobachtung, dass zu Beginn der Migräne-Attacke die Magenperistaltik vermindert ist, weisen derartige Oralia jedoch den Nachteil auf, dass die gewünschte prokinetische Wirkung von Metocolopramid nicht vollständig zum Tragen kommt und damit die Resorption von Paracetamol nicht ausreichend verbessert wird. Daher hat es sich bewährt, Paracetamol erst nach der vorherigen Gabe von Metoclopramid, vorzugsweise in einem Abstand von ca. 20 Minuten, zu applizieren. Dieses Vorgehen weist jedoch Complianceprobleme auf, da der Patient sich nicht immer strikt an die Applikationsvorschrift hält. Dies führt seinerseits zu einer verminderten Wirksamkeit des Paracetamols bzw. zu stärkeren Nebenwirkungen.

Aufgabe der vorliegenden Erfindung ist es daher, eine oral anwendbare pharmazeutische Zubereitung zu schaffen, welche die Nachteile der herkömmlichen Medikation überwindet.

Die Aufgabe wird dadurch gelöst, dass eine orale pharmazeutische Zubereitung umfassend Paracetamol und Metoclopramid in Kombination zur Verfügung gestellt wird, wobei Paracetamol im Kern und Metoclopramid in einer den Kern umgebenden Schicht umfasst ist und wobei die Zubereitung weiterhin einen mit dem Geschmackssinn wahrnehmbaren Signalstoff umfasst.

Erfindungsgemäß bevorzugt ist es dabei, dass der Signalstoff in einer Zwischenschicht zwischen Kern und der Metoclopramid umfassenden Schicht vorhanden ist.

Vorzugsweise ist es erfindungsgemäß vorgesehen, daß der Signalstoff aus pharmazeutisch unbedenklichen süß-, bitter-, sauer-, scharf-, etherisch-, aromatisch- oder salzigschmeckenden Geschmacksstoffen ausgewählt ist. Erfindungsgemäß ist es insbesondere dabei bevorzugt, dass der Geschmacksstoff eine Säure, ganz vorzugsweise eine Fruchtsäure, ist.

Die Zwischenschicht der erfindungsgemäßen orale pharmazeutische Zubereitung ist vorzugsweise ein dünn- oder dickschichtiger Überzug oder ein aufgepresster Mantel.

Erfindungsgemäß sind vorzugsweise zwischen dem Kern und der Metoclopramid umfassenden Schicht und/oder auf dieser Schicht weitere Schichten vorhanden.

Erfindungsgemäß bevorzugt ist es ferner, dass der Kern der erfindungsgemäßen oralen pharmazeutischen Zubereitung eine Kautablette ist und/oder daß das Paracetamol in mikroverkapselter und/oder pelletierter Form im Kern vorliegt.

Weiterhin ist es erfindungsgemäß bevorzugt, daß die Metoclopramid umfassende Schicht ein dünn- und dickschichtiger Überzug ist oder in Form eines Tablettenmantels vorliegt.

Ein weiterer Gegenstand der vorliegenden Erfindung ist ein Verfahren zur Herstellung einer erfindungsgemäßen oralen pharmazeutischen Zubereitung, wobei man einen Paracetamol umfassenden Kern mit einer den Signalstoff umfassenden Zwischenschicht überzieht und auf diese Zwischenschicht eine Metoclopramid umfassende Schicht aufbringt.

Alternativ kann man auf einen Paracetamol und den Signalstoff umfassenden Kern direkt eine Metoclopramid umfassende Schicht aufbringen.

Gegenstand der vorliegenden Erfindung ist auch eine pharmazeutische Packung umfassend eine erfindungsgemäße orale pharmazeutische Zubereitung und einen Verwendungshinweis, wonach die orale pharmazeutische Zubereitung solange im Mund zu lutschen ist, bis der Signalstoff wahrgenommen wird oder dessen Wahrnehmung endet und worauf die verbleibende Zubereitung dann zu Schlucken ist, wobei gegebenenfalls zuvor die Zubereitung zu zerkauen ist.

Paracetamol (CAS-Registernummer: 103-90-2) ist der internationale Freiname (INN) für das antipyretisch und schwach analgetisch wirkende 4-Acetylaminophenol (auch 4-Hydroxyacetanilid oder Acetaminophen). Paracetamol hat die Summenformel C₈H₉NO₂, die Molmasse 151,16 und bildet monokline Prismen mit einem Schmelzpunkt von 167-169 °C und einer Dichte von 1,293, ist leicht löslich in heißem Wasser und in Ethanol.

Metoclopramid (CAS-Registernummer: 364-62-5) ist der internationale Freiname (INN) für das Antiemetikum/Prokinetikum 4-Amino-5-chlor-N- [2-(diethylamino)ethyl]-2-methoxybenzamid mit der Summenformel C₁₄H₂₂ClN₃O₂, und der Molmasse 299,80. Verwendet wird meist Metoclopramid Monohydrochlorid (CAS-Registernummer: 7232-21-5) mit einem Schmelzpunkt von 182,5-184 °C, einem weißlichen, kristallinen Pulver, löslich in Wasser (1:0,7) und Ethanol (1:3) und das Metoclopramid Dihydrochlorid (CAS-Registernummer: 2576-84-3), Schmp. 145 °C (Zers.).

Die Herstellung der erfindungsgemäßen oralen pharmazeutischen Zubereitungen ist an sich bekannt und in den dem Fachmann bekannten Handbüchern beschrieben, beispielsweise Hager's Handbuch (5.) 2, 622-1045; List et al., Arzneiformenlehre, Stuttgart: Wiss. Verlagsges. 1985; Sucker et al., Pharmazeutische Technologie, Stuttgart: Thieme 1991; Ullmann's Enzyklopädie (5.) A 19, 241-271; Voigt, Pharmazeutische Technologie, Berlin: Ullstein Mosby 1995.

Die erfindungsgemäße orale pharmazeutische Zubereitung kann in unterschiedlichen Ausführungsformen vorliegen, welche alle zum Umfang der vorliegenden Erfindung gehören.

Der das Paracetamol enthaltende Kern kann in Form einer Kautablette ausgestaltet werden. Derartige Formen, bei denen für die Applikation auf die zusätzliche Einnahme von Wasser verzichtet werden kann, eignen sich in besonderer Weise für die Kupierung von plötzlich oder auf Reisen auftretenden Migräneanfällen.

Zur besonders schonenden Applikation von Paracetamol ist dieser Wirkstoff im Kern in mikroverkapselter oder pelletierter Form vorhanden. Dabei kann auch die Abgabe des Wirkstoffes über einen längeren Zeitraum in Form einer verzögerten Wirkstofffreisetzung erfolgen.

In weiteren Ausführungsformen der vorliegenden Erfindung kann der Signalstoff auch im Kern oder in der Metoclopramid umfassenden Schicht enthalten sein. Bei der Verwendung der erfindungsgemäßen Arzneiform hat der Anwender dann darauf zu achten, daß ein bestimmter Signalstoff, beispielsweise ein Vanillearoma verschwindet. In diesem Falle ist der Geschmacksstoff in der Umhüllung enthalten und das Nachlassen des Geschmacks zeigt dem Verwender an, dass nunmehr der Rest der Zubereitung zu schlucken ist.

Umgekehrt kann auch ein unangenehmer Bitterstoff wie Chinin im Kern enthalten sein, wenn dieser Kern beispielsweise zusätzlich mikroverkapseltes Paracetamol enthält. In diesem Falle wird der Verwender den Kern sogleich verschlucken, unter Trinken von Wasser und somit die Wirkung des Paracetamols einleiten.

Ist dagegen der Kern als Kautablette ausgestaltet, so kann ein angenehm empfundener Geschmacksstoff verwendet werden und dieser direkt dem Kern beigegeben werden. In diesem Falle wird der Kern bei Erreichen des angenehmen Geschmacks zerkaut und dann ohne Trinken von Wasser geschluckt.

Allerdings kann der Kern auch mit einer oder mehreren weiteren Schichten umhüllt, ummantelt und/oder überzogen sein. Dabei kann der Signalstoff in jeder beliebigen Schicht enthalten sein.

Die den Kern umgebende Schicht, welche das Metoclopramid enthält ist vorzugsweise derart ausgestaltet, dass zwischen dem Ablutschen der Metoclopramidschicht und dem Schlucken des Paracetamolkerns ein Zeitraum von vorzugsweise 20 Minuten liegt.

Alternativ kann man auch die Abgabe des applizierten Paracetamols so steuern, dass die Wirkstofffreisetzung nach 20 Minuten beginnt.

Dem Fachmann ist bekannt, wie derartige Überzüge, Umhüllungen und/oder Ummantelungen beziehungsweise verzögerte Wirkstofffreisetzungen herzustellen sind.

Zum Umfang der vorliegenden Erfindung gehören auch Zubereitungen, welche mehrere und auch verschiedene Signalstoffe in unterschiedlichen Schichten der erfindungsgemäßen oralen pharmazeutischen Zubereitungen enthalten können.

Die nachfolgenden Beispiele erläutern die Erfindung:

### Beispiel 1

Tablette mit Filmüberzug und Zwischenschicht mit Signalstoff und Schluckkern

| Zusammensetzung des Schluckkerns: | |
|---|---|
| Paracetamol | 500,00 mg |
| Povidon | 20,00 mg |
| Sorbitol | 7,00 mg |
| Hochdisperses Siliciumdioxid | 2,00 mg |
| Vorverkleisterte Stärke | 40,00 mg |
| Crosscarmellose-Natrium | 13,30 mg |
| Carmellose-Natrium | 20,05 mg |
| Mikrokristalline Cellulose | 41,00 mg |
| Magnesiumstearat | 2,00 mg |

| Zwischenschicht: | |
|---|---|
| Zitronensäure | 1,00 mg |
| Hydroxypropylmethylcellulose | 8,00 mg |
| Macrogol 400 (™) | 1,20 mg |

| Filmüberzug: | |
|---|---|
| Metoclopramid | 5,00 mg |
| Hydroxypropylmethylcellulose | 16,00 mg |
| Macrogol 400 (™) | 2,50 mg |
| Titandioxid | 3,00 mg |
| Talkum | 3,00 mg |

### Herstellung:

Paracetamol und Hochdisperses Siliciumdioxid werden im Zwangsmischer mit einer Lösung aus Povidon und Sorbitol granuliert. Das Granulat wird getrocknet, gesiebt, mit Hochdispersem Siliciumdioxid , Vorverkleisterte Stärke, Crosscarmellose-Natrium, Carmellose-Natrium und Mikrokristalline Cellulose vermischt, anschließend mit Magnesiumstearat vermischt und zu Tabletten verpreßt.

Die Tabletten werden zuerst mit der Zwischenschicht aus Zitronensäure und Macrogol 400(™), danach mit dem Filmüberzug aus Metoclopramid, Hydroxypropylmethylcellulose, Macrogol 400(™), Titandioxid und Talkum überzogen.

### Beispiel 2

Tablette mit Filmüberzug, Zwischenschicht mit Signalstoff und Kaukern

| Zusammensetzung des Kaukerns: | |
|---|---|
| Paracetamol | 500,00 mg |
| Povidon | 20,00 mg |
| Sorbitol | 270,00 mg |
| Macrogol 6000 (™) | 8,00 mg |
| Hochdisperses Siliciumdioxid | 4,00 mg |

| Zwischenschicht: | |
|---|---|
| Weinsäure | 1,00 mg |
| Hydroxypropylmethylcellulose | 8,00 mg |
| Macrogol 400 (™) | 1,20 mg |

| Filmüberzug: | |
|---|---|
| Metoclopramid | 5,00 mg |
| Hydroxypropylmethylcellulose | 16,00 mg |
| Macrogol 400 (™) | 2,50 mg |
| Titandioxid | 3,00 mg |
| Talkum | 3,00 mg |

### Herstellung:

Paracetamol, Povidon, Sorbitol, Macrogol 6000 (™) und Hochdisperses Siliciumdioxid werden in einem Faßmischer homogenisiert und direkt zu Tabletten verpreßt.

Die Tabletten werden zuerst mit der Zwischenschicht aus Weinsäure, Hydroxypropylmethylcellulose und Macrogol 400, danach mit dem Filmüberzug aus Metoclopramid, Hydroxypropylmethylcellulose, Macrogol 400(™), Titandioxid und Talkum überzogen.

### Beispiel 3

Tablette mit Filmüberzug und Schluckkern mit Signalstoff

| Zusammensetzung des Kerns: | |
|---|---|
| Paracetamol | 500,00 mg |
| Povidon | 20,00 mg |
| Sorbitol | 7,00 mg |
| Weinsäure | 5,00 mg |
| Hochdisperses Siliciumdioxid | 2,00 mg |
| Vorverkleisterte Stärke | 40,00 mg |
| Crosscarmellose-Natrium | 13,30 mg |
| Carmellose-Natrium | 20,05 mg |
| Mikrokristalline Cellulose | 41,00 mg |
| Magnesiumstearat | 2,00 mg |

| Filmüberzug: | |
|---|---|
| Metoclopramid | 5,00 mg |
| Hydroxypropylmethylcellulose | 16,00 mg |
| Macrogol 400 (™) | 2,50 mg |
| Titandioxid | 3,00 mg |
| Talkum | 3,00 mg |

### Herstellung:

Paracetamol, Weinsäure und Hochdisperses Siliciumdioxid werden im Zwangsmischer mit einer Lösung aus Povidon und Sorbitol granuliert. Das Granulat wird getrocknet, gesiebt, mit Hochdispersem Siliciumdioxid, Vorverkleisterte Stärke, Crosscarmellose-Natrium, Carmellose-Natrium und Mikrokristalline Cellulose vermischt, anschließend mit Magnesiumstearat vermischt und zu Tabletten verpreßt.

Die Tabletten werden mit einem Filmüberzug aus Metoclopramid, Hydroxypropylmethylcellulose, Macrogol 400(™), Titandioxid und Talkum überzogen.

### Beispiel 4

Tablette mit Filmüberzug und Kaukern mit Signalstoff

| Zusammensetzung des Kerns: | |
|---|---|
| Paracetamol | 500,00 mg |
| Weinsäure | 20,00 mg |
| Povidon | 20,00 mg |
| Sorbitol | 270,00 mg |
| Macrogol 6000 (™) | 8,00 mg |
| Hochdisperses Siliciumdioxid | 4,00 mg |

| Filmüberzug: | |
|---|---|
| Metoclopramid | 5,00 mg |
| Hydroxypropylmethylcellulose | 16,00 mg |
| Macrogol 400 (™) | 2,50 mg |
| Titandioxid | 3,00 mg |
| Talkum | 3,00 mg |

### Herstellung:

Paracetamol, Weinsäure, Povidon, Sorbitol, Macrogol 6000 (™) und Hochdisperses Siliciumdioxid werden in einem Faßmischer homogenisiert und direkt zu Tabletten verpreßt.

Die Tabletten werden mit dem Filmüberzug aus Metoclopramid, Hydroxypropylmethylcellulose, Macrogol 400(™), Titandioxid und Talkum überzogen.

### Beispiel 5

Manteltablette mit Schluckkern, der Signalstoff enthält

| Zusammensetzung des Kerns: | |
|---|---|
| Paracetamol | 500,00 mg |
| Weinsäure | 5,00 mg |
| Povidon | 20,00 mg |
| Maisstärke | 31,20 mg |
| Hochdisperses Siliciumdioxid | 1,60 mg |
| Magnesiumstearat | 2,60 mg |

| Mantel: | |
|---|---|
| Metoclopramid | 5,00 mg |
| Lactose-Monohydrat, tablettierbar | 50,00 mg |
| Mikrokristalline Cellulose | 150,00 mg |
| Maisstärke | 45,00 mg |
| Hochdisperses Siliciumdioxid | 0,50 mg |
| Magnesiumstearat | 1,25 mg |

### Herstellung:

Paracetamol, Weinsäure und Maisstärke werden im Zwangsmischer mit einer Lösung aus Povidon granuliert. Das Granulat wird getrocknet, gesiebt und mit Magnesiumstearat und Hochdispersem Siliciumdioxid vermischt und zu Tabletten verpreßt.

Metoclopramid, Lactose-Monohydrat, tablettierbar, Mikrokristalline Cellulose, Maisstärke und Hochdisperses Siliciumdioxid werden in einem Faßmischer homogenisiert, mit Magnesiumstearat vermischt und als Mantel direkt auf die bereits vorhandenen Kerne gepreßt.

### Beispiel 6

Manteltablette mit signalstoffhaltigem Kaukern

| Zusammensetzung des Kerns: | |
|---|---|
| Paracetamol | 500,00 mg |
| Weinsäure | 20,00 mg |
| Povidon | 20,00 mg |
| Sorbitol | 270,00 mg |
| Macrogol 6000 (™) | 8,00 mg |
| Hochdisperses Siliciumdioxid | 4,00 mg |

| Mantel: | |
|---|---|
| Metoclopramid | 5,00 mg |
| Lactose-Monohydrat, teblettierbar | 72,10 mg |
| Mikrokristalline Cellulose | 216,40 mg |
| Maisstärke | 64,90 mg |
| Hochdisperses Siliciumdioxid | 0,72 mg |
| Magnesiumstearat | 1,80 mg |

### Herstellung:

Paracetamol, Weinsäure, Povidon, Sorbitol, Macrogol 6000 (™) und Hochdisperses Siliciumdioxid werden in einem Faßmischer homogenisiert und direkt zu Tabletten verpreßt.

Metoclopramid, Lactose-Monohydrat, tablettierbar, Mikrokristalline Cellulose, Maisstärke und Hochdisperses Siliciumdioxid werden in einem Faßmischer homogenisiert, mit Magnesiumstearat vermischt und als Mantel direkt auf die bereits vorhandenen Kerne gepreßt.

### Beispiel 7

Tablette mit Schluckkern aus überzogenen signalstoffhaltigen Pellets und mit Filmüberzug

| Zusammensetzung des Kerns: | |
|---|---|
| Paracetamol | 500,00 mg |
| Weinsäure | 5,00 mg |
| Povidon | 20,00 mg |
| Sorbitol | 7,00 mg |
| Hochdisperses Siliciumdioxid | 2,00 mg |
| Eudragit RL(™) Feststoff | 20,00 mg |
| Vorverkleisterte Stärke | 40,00 mg |
| Crosscarmellose-Natrium | 13,30 mg |
| Carmellose-Natrium | 20,05 mg |
| Mikrokristalline Cellulose | 41,00 mg |
| Magnesiumstearat | 2,00 mg |

| Filmüberzug: | |
|---|---|
| Metoclopramid | 5,00 mg |
| Hydroxypropylmethylcellulose | 16,00 mg |
| Macrogol 400 (™) | 2,50 mg |
| Titandioxid | 3,00 mg |
| Talkum | 3,00 mg |

### Herstellung:

Paracetamol und Hochdisperses Siliciumdioxid werden im Zwangsmischer mit einer Lösung aus Povidon und Sorbitol granuliert. Das Granulat wird gerundet und mit Eudragit RL (™) 12,5 überzogen, getrocknet und mit Vorverkleisterter Stärke, Croocarmmellose-Natrium, Carmellose-Natrium und Mikrokristalline Cellulose vermischt, anschließend mit Magnesiumstearat vermischt und zu Tabletten verpreßt.

Die Tabletten werden danach mit dem Filmüberzug aus Metociopramid, Hydroxypropylmethylcellulose, Macrogol 400 (™), Titandioxid und Talkum überzogen.

### Beispiel 8

Tablette mit Schluckkern aus überzogenen signalstoffhaltigen Pellets, mit signalstoffhaltiger Zwischenschicht und Filmüberzug

| Zusammensetzung des Kerns: | |
|---|---|
| Paracetamol | 500,00 mg |
| Weinsäure | 5,00 mg |
| Povidon | 20,00 mg |
| Sorbitol | 7,00 mg |
| Hochdisperses Siliciumdioxid | 2,00 mg |
| Eudragit RL (™) Feststoff | 20,00 mg |
| Vorverkleisterte Stärke | 40,00 mg |
| Crosscarmellose-Natrium | 13,30 mg |
| Carmellose-Natrium | 20,05 mg |
| Magnesiumstearat | 2,00 mg |
| Mikrokristalline Cellulose | 41,00 mg |

| Zwischenschicht: | |
|---|---|
| Weinsäure | 1,00 mg |
| Hydroxypropylmethylcellulose | 8,00 mg |
| Macrogol 400 (™) | 1,00 mg |

| Filmüberzug: | |
|---|---|
| Metoclopramid | 5,00 mg |
| Hydroxypropylmethylcellulose | 16,00 mg |
| Macrogol 400 (™) | 2,50 mg |
| Titandioxid | 3,00 mg |
| Talkum | 3,00 mg |

### Herstellung:

Paracetamol und Hochdisperses Siliciumdioxid werden im Zwangsmischer mit einer Lösung aus Povidon und Sorbitol granuliert. Das Granulat wird gerundet und mit Eudragit RL 12,5 überzogen, getrocknet und mit Vorverkleisterte Stärke, Crosscarmellose-Natrium, Carmellose-Natrium und Mikrokristalline Cellulose vermischt, anschließend mit Magnesiumstearat vermischt und zu Tabletten verpreßt.

Die Tabletten werden zuerst mit der Zwischenschicht aus Weinsäure, Hydroxypropylmethylcellulose und Macrogol 400 (™), danach mit dem Filmüberzug aus Metoclopramid, Hydroxypropylmethylcellulose, Macrogol 400 (™), Titandioxid und Talkum überzogen.

### Beispiel 9

Tablette mit Schluckkern aus mikroverkapseltem Paracetamol und Signalstoff, mit Filmüberzug

| Zusammensetzung des Kerns: | |
|---|---|
| Paracetamol | 500,00 mg |
| Sorbitol | 7,00 mg |
| Eudragit RL (™) Feststoff | 20,00 mg |
| Weinsäure | 5,00 mg |
| Hochdisperses Siliciumdioxid | 2,00 mg |
| Povidon | 20,00 mg |
| Vorverkleisterte Stärke | 40,00 mg |
| Crosscarmellose-Natrium | 13,30 mg |
| Carmellose-Natrium | 20,05 mg |
| Mikrokristalline Cellulose | 41,00 mg |
| Magnesiumstearat | 2,00 mg |

| Filmüberzug: | |
|---|---|
| Metoclopramid | 5,00 mg |
| Hydroxypropylmethylcellulose | 16,00 mg |
| Macrogol 400 (™) | 2,50 mg |
| Titandioxid | 3,00 mg |
| Talkum | 3,00 mg |

### Herstellung:

Paracetamol, Sorbitol und Eudragit RL (™) 12,5 werden mikroverkapselt und in einem Faßmischer mit Weinsäure, Povidon, Hochdispersesm Siliciumdioxid, Vorverkleisterte Stärke, Crosscarmellose-Natrium, Carmellose-Natrium und Mikrokristalline Cellulose vermischt, anschließend mit Magnesiumstearat vermischt und zu Tabletten verpreßt.

Die Tabletten werden mit einem Filmüberzug aus Metoclopramid, Hydroxypropylmethylcellulose, Macrogol 400 (™), Titandioxid und Talkum überzogen.

### Beispiel 10

Tablette mit Schluckkern aus mikroverkapseltem Paracetamol, mit Zwischenschicht mit Signalstoff und Filmüberzug

| Zusammensetzung des Kerns: | |
|---|---|
| Paracetamol | 500,00 mg |
| Sorbitol | 7,00 mg |
| Eudragit RL (™) Feststoffe | 20,00 mg |
| Hochdisperses Siliciumdioxid | 2,00 mg |
| Povidon | 20,00 mg |
| Vorverkleisterte Stärke | 40,00 mg |
| Crosscarmellose-Natrium | 13,30 mg |
| Carmellose-Natrium | 20,05 mg |
| Mikrokristalline Cellulose | 41,00 mg |
| Magnesiumstearat | 2,00 mg |

| Zwischenschicht: | |
|---|---|
| Weinsäure | 1,00 mg |
| Hydroxypropylmethylcellulose | 8,00 mg |
| Macrogol 400 (™) | 1,20 mg |

| Filmüberzug: | |
|---|---|
| Metoclopramid | 5,00 mg |
| Hydroxypropylmethylcellulose | 16,00 mg |
| Macrogol 400 (™) | 2,50 mg |
| Titandioxid | 3,00 mg |
| Talkum | 3,00 mg |

### Herstellung:

Paracetamol, Sorbitol und Eudragit RL (™) 12,5 werden mikroverkapselt und in einem Faßmischer mit Povidon, Hochdispersem Siliciumdioxid, Vorverkleisterter Stärke, Crosscarmellose-Natrium, Carmellose-Natrium und Mikrokristalline Cellulose homogenisiert, anschließend mit Magnesiumstearat vermischt und zu Tabletten verpreßt.

Die Tabletten werden zuerst mit der Zwischenschicht aus Weinsäure, Hydroxypropylmethylcellulose und Macrogol 400, danach mit dem Filmüberzug aus Metoclopramid, Hydroxypropylmethylcellulose, Macrogol 400, Titandioxid und Talkum überzogen.

## Patentansprüche

1. Orale pharmazeutische Zubereitung umfassend Paracetamol und Metoclopramid in Kombination, **dadurch gekennzeichnet, dass** Paracetamol im Kern und Metoclopramid in einer den Kern umgebenden Schicht umfasst ist und wobei die Zubereitung weiterhin einen mit dem Geschmackssinn wahrnehmbaren Signalstoff umfasst.

2. Orale pharmazeutische Zubereitung gemäß Anspruch 1, **dadurch gekennzeichnet, daß** der Signalstoff in einer Zwischenschicht zwischen Kern und der Metoclopramid umfassenden Schicht vorhanden ist.

3. Orale pharmazeutische Zubereitung gemäß einem der voranstehenden Ansprüche, **dadurch gekennzeichnet, dass** der Signalstoff aus pharmazeutisch unbedenklichen süß-, bitter-, sauer-, scharf-, ätherisch-, aromatisch- oder salzigschmeckenden Geschmacksstoffen ausgewählt ist.

4. Orale pharmazeutische Zubereitung gemäß einem der voranstehenden Ansprüche, **dadurch gekennzeichnet, dass** der Geschmacksstoff eine Säure, vorzugsweise eine Fruchtsäure, ist.

5. Orale pharmazeutische Zubereitung gemäß einem der voranstehenden Ansprüche, **dadurch gekennzeichnet, dass** die Zwischenschicht ein dünn- oder dickschichtiger Überzug oder ein aufgepresster Mantel ist.

6. Orale pharmazeutische Zubereitung gemäß einem der voranstehenden Ansprüche, **dadurch gekennzeichnet, dass** zwischen dem Kern und der Metoclopramid umfassenden Schicht und/oder auf dieser Schicht weitere Schichten vorhanden sind.

7. Orale pharmazeutische Zubereitung gemäß einem der voranstehenden Ansprüche, **dadurch gekennzeichnet, dass** der Kern eine Kautablette ist und/oder das Paracetamol in mikroverkapselter oder pelletierter Form im Kern vorliegt.

8. Orale pharmazeutische Zubereitung gemäß einem der voranstehenden Ansprüche, **dadurch gekennzeichnet, dass** die Metoclopramid umfassende Schicht ein dünnoder dickschichtiger Überzug ist oder in Form eines Tablettenmantels vorliegt.

9. Orale pharmazeutische Zubereitung gemäß Anspruch 1, **dadurch gekennzeichnet, dass** der Signalstoff in der Paracetamol umfassenden Schicht ist.

10. Verfahren zur Herstellung einer oralen pharmazeutische Zubereitung gemäß Anspruch 1, **dadurch gekennzeichnet, dass** man einen Paracetamol umfassenden Kern mit einer den Signalstoff umfassenden Zwischenschicht überzieht und auf diese Zwischenschicht eine Metoclopramid umfassende Schicht aufbringt.

11. Pharmazeutische Packung umfassend eine orale pharmazeutische Zubereitung gemäß Anspruch 1 und einen Verwendungshinweis, wonach die orale pharmazeutische Zubereitung solange im Mund zu lutschen ist, bis der Signalstoff wahrgenommen wird oder dessen Wahrnehmung endet und worauf die verbleibende Zubereitung dann zu Schlucken ist, wobei gegebenenfalls zuvor die Zubereitung zu zerkauen ist.

## Claims

1. Oral pharmaceutical preparation containing paracetamol and metoclopramide in combination, **characterised in that** paracetamol is contained in the core and metoclopramide is contained in a layer surrounding the core and the preparation also contains a signal substance which is detectable by taste.

2. Oral pharmaceutical preparation according to claim 1, **characterised in that** the signal substance is present in an intermediate layer between the core and the layer containing the metoclopramide.

3. Oral pharmaceutical preparation according to one of the preceding claims, **characterised in that** the signal substance is selected from pharmaceutically safe sweet-, bitter-, sour-, sharp-, ethereal-, aromatic- or salty-tasting flavourings.

4. Oral pharmaceutical preparation according to one of the preceding claims, **characterised in that** the flavouring is an acid, preferably a fruit acid.

5. Oral pharmaceutical preparation according to one of the preceding claims, **characterised in that** the intermediate layer is a thin or thick coating or a pressed-on covering.

6. Oral pharmaceutical preparation according to one of the preceding claims, **characterised in that** between the core and the layer containing the metoclopramide and/or on this layer are provided additional layers.

7. Oral pharmaceutical preparation according to one of the preceding claims, **characterised in that** the core is a chewable tablet and/or the paracetamol is present in the core in a microencapsulated or pelleted form.

8. Oral pharmaceutical preparation according to one of the preceding claims, **characterised in that** the layer containing the metoclopramide is a thin or thick coating or takes the form of a tablet coating.

9. Oral pharmaceutical preparation according to claim 1, **characterised in that** the signal substance is present in the layer containing the paracetamol.

10. Process for preparing an oral pharmaceutical preparation according to claim 1, **characterised in that** a core containing paracetamol is coated with an intermediate layer containing the signal substance and over this intermediate layer is applied a layer containing metoclopramide.

11. Pharmaceutical package comprising an oral pharmaceutical preparation according to claim 1 and instructions for use, according to which the oral pharmaceutical preparation is to be sucked in the mouth until the signal substance is detected or ceases to be detected and thereafter the remainder of the preparation is to be swallowed, the preparation optionally being chewed beforehand.

## Revendications

1. Préparation orale pharmaceutique comprenant du paracétamol et du métoclopramide en combinaison,
**caractérisée en ce que**
le paracétamol est inclus dans le noyau et le métoclopramide dans une couche qui entoure le noyau, et dans lequel la préparation inclut en outre une substance de signalisation perceptible dans le sens du goût.

2. Préparation orale pharmaceutique conformément à la revendication 1,
**caractérisée en ce que**
la substance de signalisation est présente dans une couche intermédiaire entre le noyau et la couche qui entoure le métoclopramide.

3. Préparation orale pharmaceutique conformément à l'une quelconque des revendications précédentes,
**caractérisée en ce que**
la substance de signalisation est choisie parmi les substances de sapidité pharmaceutiquement inoffensives qui donnent une saveur douce, amère, acide, épicée, éthérée, aromatique ou saline.

4. Préparation orale pharmaceutique conformément à l'une quelconque des revendications précédentes,
**caractérisée en ce que**
la substance de sapidité est un acide, en particulier un acide de fruit.

5. Préparation orale pharmaceutique conformément à l'une quelconque des revendications précédentes,
**caractérisée en ce que**
la couche intermédiaire est une pellicule en couche mince ou en couche épaisse ou une enveloppe imprimée.

6. Préparation orale pharmaceutique conformément à l'une quelconque des revendications précédentes,
**caractérisée en ce que**
la couche entre le noyau et celle incluant le métoclopramide et/ou sur cette couche, d'autres couches sont présentes.

7. Préparation orale pharmaceutique conformément à l'une quelconque des revendications précédentes,
**caractérisée en ce que**
le noyau est un comprimé à mâcher et/ou **en ce que** le paracétamol se présente sous forme micro-encapsulée ou sous forme de pellets.

8. Préparation orale pharmaceutique conformément à l'une quelconque des revendications précédentes,
**caractérisée en ce que**
la couche qui inclut le métoclopramide est une pellicule à couche mince ou épaisse, ou se présente sous forme d'une enveloppe de comprimé.

9. Préparation orale pharmaceutique conformément à la revendication 1,
**caractérisée en ce que**
la substance de signalisation est dans la couche qui inclut le paracétamol.

10. Procédé de production d'une préparation orale pharmaceutique conforme à la revendication 1,
**caractérisé en ce qu'**
on revêt un noyau incluant du paracétamol avec une couche intermédiaire incluant la substance de signalisation et on applique sur cette couche intermédiaire une couche incluant le métoclopramide.

11. Emballage pharmaceutique contenant une préparation orale pharmaceutique conforme à la revendication 1, et une notice d'information d'utilisation, selon laquelle on doit sucer la préparation orale pharmaceutique aussi longtemps dans la bouche jusqu'à ce que la substance de signalisation soit perçue ou que sa perception se termine, et après quoi on doit ensuite déglutir la préparation restante, pour laquelle éventuellement au préalable on doit broyer en mâchant la préparation.
